# EUROPÄISCHE PATENTANMELDUNG

(11) **EP 1 079 016 A2**
(43) Veröffentlichungstag der Anmeldung: **28.02.2001**
(21) Anmeldenummer: 00116987.9
(22) Anmeldetag: 08.08.2000
(51) Int. Cl.: D06N 3/12, D06N 7/00

(54) **Rutschhemmende Beschichtung auf den Körper bedeckende Trägermaterialien sowie Verwendung derselben**

(30) Priorität: 24.08.1999 DE 19940019
(71) Anmelder: Beiersdorf Aktiengesellschaft, 20245 Hamburg (DE)
(72) Erfinder: Andrews, Arthur-Hugh, 25337 Kölln-Reisiek (DE); Bodenschatz, Stefan, Dr., 21614 Buxtehude (DE); Himmelsbach, Peter, 21614 Buxtehude (DE); Jauchen, Peter, 22455 Hamburg (DE)

(57) **Zusammenfassung**

Rutschhemmende Beschichtung zur zumindest partiellen Aufbringung auf den Körper bedeckende Trägermaterialien, beinhaltend ein Polyaddukt mit einer Phase 1 und einer Phase 2, wobei der Masseanteil von Phase 1 größer 60 Gew.-% ist,
Phase 1 ein Polyadditionsprodukt aus mindestens einem Edukt E1 und mindestens einem Edukt E3 ist, wobei Edukt E1 ein oligomeres Diol darstellt, welches eine Hydroxylzahl von mehr als 20 mg KOH/g (Diol) aufweist, und wobei Edukt E3 ein silikonfreies, zumindest bifunktionelles Reaktionsedukt mit einem Molekulargewicht von weniger als 300 g/mol ist,
Phase 2 ein Polyadditionsprodukt aus mindestens einem Edukt E2 und mindestens einem Edukt E3 ist, wobei das Edukt E2 wenigstens teilweise endständige funktionelle Gruppen aufweist und
die Beschichtung ein Stoffmengenverhältnis der zu vernetzenden Edukte E3 zu der Summe aus einem oder mehreren oligomeren Diolen (Edukt E1) und dem Anteil weiterer Edukte E2 einen Quotienten von 0,9 bis 1,1 aufweist.

## Beschreibung

Die Erfindung betrifft eine rutschhemmende Beschichtung auf den Körper bedeckende oder anatomisch ausgeformte Trägermaterialien sowie Verwendung derselben in Bandagen und Strümpfen, insbesondere Antithrombosestrümpfen.

Medizinische Strümpfe sind bekannt. Sie verbessern aufgrund ihrer kompressiven Eigenschaften die venöse Strömungsgeschwindigkeit. Für immobilisierte Patienten werden Strümpfe prophylaktisch zur Vorbeugung von Embolien oder Thrombosen eingesetzt.

Rutschhemmende oder -verhindernde Ausrüstungen für derartige Strümpfe sind ebenfalls bekannt. Insbesondere werden Antirutschbeschichtungen für den Sohlenbereich oder den Kantenbereich an der Strumpföffnung beschrieben.

Im Stand der Technik basieren diese Beschichtungen auf natürlichen und synthetischen Polymeren. Insbesondere die Verwendung von Amid-, Silikon-, Acrylat-, Vinyl- und Styrolverbindungen, aber auch die Verwendung von Kautschuk und Latex sind bekannt.

Nachteilig bei diesen Verbindungen ist zum einen die geringe Waschfestigkeit bei höheren Temperaturen oder die eingeschränkte Haftung auf dem Strumpf. Zum anderen sind chemisch reaktive Verbindungen schwierig in der Herstellung oder hinsichtlich der Verträglichkeit, insbesondere im Kontakt mit der Haut, bedenklich. Weiter sind waschbeständige Beschichtungen hart und wenig anschmiegsam.

Marktgängige Produkte an Strümpfen enthalten einen rutschhemmenden Rand an der oberen Öffnung des Strumpfes, welcher separat angebracht, häufig angenäht worden ist.
Dies ist nicht nur unvorteilhaft hinsichtlich der Kosten, sondern auch für das Handling in der Fertigung. Vorteilhaft ist jedoch die Flexibilität in der Produktgestaltung sowie die Festigkeit des Verbundes. Weiter bildet dieser Rand, welcher in der Regel aus einem anderen Material besteht, einen Schutz gegen das Umschlagen der Kante.

US 3,983,870 offenbart eine rutschfeste Unterstützung für Gliedmassen, insbesondere einen medizinischen Strumpf. Zur Herstellung der rutschverhindernden Beschichtung kommen u.a. Acrylate und Kautschuke zum Einsatz. In einer weiteren Aufzählung von Stoffen, ohne eine Angabe einer möglichen Lehre, sind undifferenziert duromere Urethane erwähnt, nicht hingegen offenbart werden vernetzte oligomere Diole.

DE 39 16 040 beschreibt ein Verfahren zum Aufbringen von rutschhemmenden Massen auf Kleidungsstücken, wobei die Kleidungsstücke umgestülpt werden. Anschließend läßt man diese um ihre Längsachse rotieren. Insbesondere werden hier reaktive Silikon-Kautschuke zur Bildung der rutschhemmenden Massen eingesetzt. Aufgrund der Reaktivität ist das Verfahren sehr aufwendig, da Silikone gehärtet werden müssen.

In US 3,728,875 wird ein Strumpf mit einem inneren weichen Bereich beschrieben, wobei ein elastisches Band auf Urethanbasis angenäht wird. Aus der Lehre geht nicht hervor, ob es sich dabei um vernetzte oligomere Diole handelt. Ferner ist das Anbringen eines solchen Bandes teuer, so daß es gegenüber dem einfachen Ausrüsten mittels Beschichten unvorteilhaft ist.

WO 97/45081 offenbart einen Strumpf für medizinische Zwecke mit einem besonderen Polyurethanfaden zur Kompressionserzeugung . Durch die Verwendung dieser Fäden wird keine rutschhemmende Wirkung beschrieben.

Aufgabe der Erfindung ist es, eine rutschhemmende Beschichtung zur Verfügung zu stellen, die für medizinische Anforderungen geeignet ist und die die aus dem Stand der Technik bekannten Nachteile nicht aufweist.

Gelöst wird die Aufgabe durch eine Beschichtung, wie sie im Hauptanspruch dargelegt ist. Die Unteransprüche umfassen vorteilhafte Varianten des Erfindungsgegenstands sowie Anwendungen derselben.

Demgemäß betrifft die Erfindung eine silikonfreie, nicht klebende rutschhemmende beziehungsweise rutschverhindernde Beschichtung zur zumindest partiellen Aufbringung auf den Körper bedeckende Trägermaterialien, beinhaltend ein Polyaddukt mit einer Phase 1 und einer Phase 2, wobei der Masseanteil von Phase 1 größer 60 Gew.-% ist.
Phase 1 ist ein Polyadditionsprodukt aus mindestens einem Edukt E1 und mindestens einem Edukt E3, wobei Edukt E1 ein oligomeres Diol darstellt, welches eine Hydroxylzahl von mehr als 20 mg KOH/g (Diol) aufweist, und wobei Edukt E3 ein silikonfreies, zumindest bifunktionelles Reaktionsedukt mit einem Molekulargewicht von weniger als 300 g/mol ist.
Phase 2 ist ein Polyadditionsprodukt aus mindestens einem Edukt E2 und mindestens einem Edukt E3, wobei das Edukt E2 wenigstens teilweise endständige funktionelle Gruppen aufweist.
Die Beschichtung weist dabei ein Stoffmengenverhältnis der zu vernetzenden Edukte E3 zu der Summe aus einem oder mehreren oligomeren Diolen (Edukt E1) und dem Anteil weiterer Edukte E2 einen Quotienten von 0,9 bis 1,1 auf, bevorzugt zwischen 0,91 und 1,05 und besonders bevorzugt zwischen 0,92 und 1,02.

Nach Römpp (Römpp Lexikon Chemie - Version 1.5, Stuttgart/New York: Georg Thieme Verlag 1998) beschreibt die Hydroxylzahl eine Maßzahl, die angibt, wieviel Milligramm Kaliumhydroxid der Essigsäure-Menge äquivalent sind, die von 1 g Substanz bei der Acetylierung gebunden wird. Die Probe wird im allg. mit Essigsäureanhydrid-Pyridin gekocht und die entstehende Säure mit KOH-Lösung titriert.
Die Hydroxylzahl, die zur Beurteilung von Reaktionsharzen, Wachsen, Fetten, Ölen, Lösungsmitteln usw. dient, ist verwandt, in angloamerikanischen Untersuchungen identisch mit der Acetylzahl.
Weiterführende Angaben lassen sich in den DIN-Normen 53 240 (12/1971) und 53 240-2 (12/1993) finden.

In einer bevorzugten Ausführungsform ist das Verhältnis des Masseanteils von Phase 1 und Phase 2 größer 1,5, in weiteren vorteilhaften Ausführungen zwischen 1,8 und 100, besonders vorteilhaft 2,0 bis 20.
Weiter vorzugsweise ist der Masseanteil von Phase 1 zwischen 67 Gew.-% und 98 Gew.-%. Besonders vorteilhaft sind Polyaddukte mit Gewichtsanteil von 75 Gew.-% bis 98 Gew.-% in der Phase 1, besonders geeignet 76 Gew.-% bis 95 Gew.-%.

Eine abgewandelte funktionsgerechte rutschhemmende Beschichtung wird durch das Schäumen derselben erreicht.

Die zu verwendenden rustschhemmenden Substanzen werden dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.-% betragen und kann bis zu etwa 85 Vol.-% reichen. In der Praxis haben sich Werte von 10 Vol.-% bis 75 Vol.-%, bevorzugt 20 bis 60 Vol.-%, besonders bevorzugt 40 bis 60 Vol.-% gut bewährt.
Wird bei relativ hohen Temperaturen von ungefähr 100 °C bis 240 °C und vergleichsweise hohem Innendruck gearbeitet, können sehr offenporige Antirutschschaumschichten entstehen, die besonders gut luft- und wasserdampfdurchlässig sind.
Die vorteilhaften Eigenschaften der geschäumten Beschichtung sind die gute Anschmiegsamkeit auch an unebenen Flächen durch die Elastizität und Plastizität der geschäumten Vorrichtung.

Ein besonders geeignetes Verfahren zur Herstellung der erfindungsgemäß geschäumten rustschhemmenden Ausrüstung arbeitet nach dem Schaum-Mix-System. Hierbei wird die thermoplastische Antirutschmasse unter hohem Druck bei einer Temperatur über dem Erweichungspunkt (ungefähr 180 °C) mit den vorgesehenen Gasen wie zum Beispiel Stickstoff, Luft oder Kohlendioxid in unterschiedlichen Volumenanteilen (etwa 10 Vol.-% bis 80 Vol.-%) in einem Stator/Rotorsystem umgesetzt.

Während der Gasvordruck größer 100 bar ist, betragen die Mischdrucke Gas/Thermoplast im System 40 bis 100 bar, bevorzugt 40 bis 70 bar. Der so hergestellte Antirutschschaum kann anschließend über eine Leitung in das Auftragswerk gelangen. Bei dem Auftragswerk finden handelsübliche Düsen-, Extruder- oder Kammersysteme Verwendung.
Durch die Schäumung der Beschichtung und die dadurch entstandenen offenen Poren in der Masse sind bei Verwendung eines an sich porösen Trägers die mit der rutschhemmenden Ausrüstung beschichteten Produkte gut wasserdampf- und luftdurchlässig.
Die benötigte Antirutschmittelmenge wird erheblich reduziert ohne Beeinträchtigung und Wirkungsweise der Eigenschaften.

Vorteilhaft insbesondere für die Verwendung der rutschhemmenden Beschichtung bei medizinischen Produkten ist weiterhin, wenn die Masse partiell auf dem Trägermaterial aufgetragen ist, beispielsweise durch Rasterdruck, Thermosiebdruck, Thermoflexodruck oder Tiefdruck, denn im Vollstrich ausgerüstete Trägermaterialien können unter ungünstigen Voraussetzungen bei der Applikation mechanische Hautirritationen hervorrufen.

Ferner kann die rutschhemmende Ausrüstung beispielsweise auch aufgesprüht oder versponnen werden, was ein mehr oder weniger unregelmäßiges Auftragsbild ergibt.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien.
Hierdurch werden Hautirritationen, die durch Stauungen der Körperflüssigkeiten hervorgerufen werden, vermieden. Die angelegten Abführungskanäle ermöglichen ein Ableiten.

Bevorzugt wird der Auftrag in Form von polygeometrischen Kalotten und ganz besonders von solchen Kalotten, bei denen das Verhältnis Durchmesser zu Höhe kleiner 5:1 ist.
Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.
Die rutschhemmende Substanz kann gleichmäßig auf dem Trägermaterial verteilt sein, sie kann aber auch funktionsgerecht für das Produkt über die Fläche unterschiedlich stark oder dicht aufgetragen sein.

Das Prinzip des Thermosiebdrucks besteht in der Verwendung einer rotierenden beheizten, nahtlosen, trommelförmigen perforierten Rundschablone, die über eine Düse mit der bevorzugten Masse beschickt wird. Eine speziell geformte Düsenlippe (Rund- oder Vierkantrakel) preßt die über einen Kanal eingespeiste Masse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Kalotten geschieht dabei nach folgendem Mechanismus:

Der Düsenrakeldruck fördert die Masse durch die Siebperforation an das Trägermaterial.
Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die Adhäsion der Antirutschmasse in der Schmelze und die innere Kohäsion des Hotmelts wird von der auf dem Träger bereits haftenden Basis der Kalotten der in den Löchern begrenzte Vorrat an rutschhemmender Substanz konturenscharf abgezogen beziehungsweise durch den Rakeldruck auf den Träger gefördert.
Nach Beendigung dieses Transportes formt sich, abhängig von der Rheologie der rutschhemmenden Masse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Masse ab.

Bei der Siebschablone im Thermosiebdruck kann das Steg/Loch-Verhältnis kleiner 10:1 sein, bevorzugt kleiner oder gleich 1:1, insbesondere gleich 1:10.

Der beschriebene Bildungsmechanismus der Kalotten erfordert bevorzugt saugfähige oder zumindest von der kohäsiven Masse benetzbare Trägermaterialien.

Schwierig zu benetzende Strumpfoberflächen müssen durch chemische oder physikalische Verfahren vorbehandelt werden. Dies kann durch zusätzliche Maßnahmen wie zum

Beispiel Coronaentladung oder Beschichtung mit die Benetzung verbessernden Stoffen geschehen.

Mit dem aufgezeigten Druckverfahren können die Größe und Form der Kalotten definiert festgelegt werden. Der Basisdurchmesser der Kalotten kann von 10 µm bis 10000 µm gewählt werden, die Höhe der Kalotten von 20 µm bis 2000 µm, bevorzugt 50 µm bis 1000 µm, wobei der Bereich kleiner Durchmesser für glatte Träger, der mit größerem Durchmesser und größerer Kalottenhöhe für rauhe oder stark porige Trägermaterialien vorgesehen ist.
Die Positionierung der Kalotten auf dem Träger wird durch die in weiten Grenzen variierbare Geometrie des Auftragswerkes, zum Beispiel Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 220 m/min, beschichtet, wobei die Beschichtungstemperatur größer als die Erweichungstemperatur zu wählen ist.

Die rutschhemmende oder rutschverhindernde Masse kann mit einem Flächengewicht von größer 3 g/m², bevorzugt zwischen 6 g/m² und 1000 g/m², ganz besonders bevorzugt zwischen 9 g/m² und 750 g/m², auf dem Trägermaterial aufgetragen sein. Für eine spezielle Strumpfausrüstung sind Flächengewichte von 300 g/m² bis 700 g/m² notwendig.
Hier werden kleine Ausschnitte für den Strumpfrand mit einer hohen Biegesteifigkeit, insbesondere einer breitenbezogenen Biegesteifigkeit gemäß DIN 53121, genutzt.

Der prozentuale Anteil, der mit der Masse partiell beschichteten Fläche sollte mindestens 5 % betragen und kann bis zu ungefähr 95 % reichen, für spezielle Produkte bevorzugt 20 % bis 60 % sowie 70 % bis 95 %. Dieses kann gegebenenfalls durch Mehrfachapplikation oder spezielle Verformungsschritte erreicht werden, wobei gegebenenfalls auch Massen mit unterschiedlichen Eigenschaften eingesetzt werden können. Bei speziellen Strumpfanwendung werden 10 bis 50 % des Beschichtungsbereichs ausgerüstet.

Als rutschhemmende Ausrüstung dienen Stoffe auf thermoplastischer Basis, welche sich durch gute Waschbarkeit, Hautverträglichkeit und Thermostablität auszeichnen.

Vorteilhaft sind Stoffe, welche keinen oder wenig Masseschwund durch das Waschen mit herkömmlichen Waschmitteln erleiden. Die eingesetzten Stoffe weisen zumindest bei einer Waschtemperatur im Bereich von 5 bis 96 °C beziehungsweise bei Temperaturen um und über 100 °C einen Masseschwund von weniger als 10 % nach mindestens fünf Waschläufen auf.
Die rutschhemmende Beschichtung sollte also ganz besonders im Temperaturbereich von 5 °C bis 100 °C hydrolysebeständig sein.

Für eine anschmiegsame, rutschhemmende oder -verhindernde Ausrüstung ist der Einsatz von wenigstens teilweise linearen Polymeren auf Basis vernetzter oligomerer Diole (E1) vorteilhaft, und zwar weil unvernetzte oligomere Diole wenig oder gar nicht waschbeständig sind. Eine rutschhemmende oder rutschverhindernde Wirkung kann bei unvernetzten Diolen darüber hinaus auch nicht festgestellt werden.
Das einzusetzende oligomere Diol ist in der Regel ein Syntheseprodukt, welches durch Polyaddition zu einem rutschhemmenden Material umgesetzt wird. Für die spezielle Verwendung als rutschhemmende oder -hindernde Substanz sind oligomere Diole mit einer Hydroxylzahl (DIN 53240) von mindestens 20 notwendig, bevorzugt 40 bis 230 mg KOH/g, besonders bevorzugt 65 bis 224 mg KOH/g, ganz besonders bevorzugt 70 bis 150 mg KOH/g. Bei der Auswahl der Diole ist auf die ausreichende Hydrolysebeständigkeit zu achten. Polyether, spezielle Polyester, Polyacryl- und Methacrylsäureverbindungen sind verwendbar. Generell sind Polyether auf Basis von Polyethylenoxiddiol, Polypropylenoxiddiol und Polytetramethylenoxiddiol vorteilhaft.

Vertreter der zweiten Eduktgruppe (E2) sind niedermolekulare Diole oder Diamine. Hier sind das Ethandiol, Butandiol und Hexandiol, aber auch diverse aliphatische Diamine zu nennen. Eine jeweils endständige Bindung der Hydroxyl- oder Aminogruppe ist ebenfalls vorteilhaft.
Vorzugsweise ist das Edukt E2 ein 1,2-Ethandiol, 1,4 Butandiol, 1,6 Hexandiol und/oder eine Mischung aus den vorgenannten.

Vertreter der dritten Eduktgruppe (E3) haben ebenfalls, wenigstens teilweise, endständige funktionelle Gruppen. Besonders bevorzugt für die Vernetzung ist der Einsatz von einem oder mehreren silikonfreien bifunktioneflen Reaktionsedukten mit einem Molekulargewicht von weniger als 300 g/mol in der Ausgangsform. Wichtigste Vertreter dieser Gruppe sind epoxid- und isocyanathaltige Verbindungen. Vorzugsweise ist das Edukt E3 ein aliphatisches oder aromatisches Diisocyanat.

Bei einer weiteren bevorzugten Ausführungsform beinhaltet die Beschichtung weniger als 5 Gew.-% Allophanatgruppen, bevorzugt 0,01 bis 2 Gew.-%.

Je nach Verwendung können der rutschhemmenden Beschichtung initiatorisch, katalysatorisch oder stabilisierend wirkende Substanzen beigefügt werden.

Um einen zusätzlichen Effekt zu erzielen, können der Beschichtung auch Wirkstoffe zugesetzt werden, und zwar vorzugsweise in Mengenkonzentrationen des oder der Wirkstoffe zwischen 0,01 bis 50 Gew.-%, vorzugsweise 0,1 bis 20 Gew.-%.

Unter der Bezeichnung 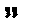 Wirkstoffe" im Zusammenhang mit der vorliegenden Erfindung werden chemische Elemente, organische und anorganische Verbindungen verstanden, die aus den sie enthaltenden Bestandteilen einer gattungsgemäßen Beschichtung herauswandern können und dadurch einen angestrebten Effekt hervorrufen. Unter den Anwendungsgebieten der erfindungsgemäßen Beschichtung ist die Human- und Tiermedizin von besonderer Bedeutung.
Typische verabreichbare Stoffe sind hierbei:

Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, L-Äpfelsäure, Alimemazin, Allithiamin, Allyl Isothiocyanat, Aminoethanol, Apyzin, Apiol, Azatadin, Alprenolol, Äthinazon, Benzoylperoxid, Benzylalkohol, Bisabolol, Bisnorephedrin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Diethylethan, Divinylethan, Dexchlorpheniramin, Dinoproston, Dixyrazin, Ephedrin, Ethosuximid, Enallylpropymal, Emylcamat, Erytroltetranitrat, Emetin, Enfluran, Eucalyptol, Etofenamat, Ethylmorphin, Fentanyl, Fluanison, Guajazulen, Halothan, Hyoscyamin, Histamin, Fencarbamid, Hydroxycain, Hexylresorcin, Isoaminilcitrat, Isosorbiddinitrat, Ibuprofen, Jod, Jodoform, Isoaminil, Lidocain, Lopinn, Levamisol, Methadon, Methyprylon, Methylphenidat, Mephenesin, Methylephedrin, Meclastin, Methopromazin, Mesuximid, Nicethamid, Norpseudoephedrin, Menthol, Methoxyfluran, Methylpentinol, Metixen, Mesoprostol, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäureester, Spartein, Trichlorethylen, Timolol, Trifluperazin, Tetracain, Trimipramin, Tranylcypromin, Trimethadion, Tybamat, Thymol, Thioridazin, Valproinsäure, Verapamil, sowie weitere, dem Fachmann geläufige, über die Haut aufnehmbare Wirkstoffe. Selbstverständlich ist diese Aufzählung nicht abschließend.

Die Verteilung der Wirkstoffe in der Beschichtung kann in einem Thermohomogenisator wie zum Beispiel Thermomixer, Thermokneter, Walzenwerke oder Schneckensysteme durchgeführt werden. Die Zugabe des Wirkstoffs kann in die vollständig hergestellte Beschichtung erfolgen, ebenso gut kann dieser in eine Zwischenstufe oder in die Ausgangsmischung eingearbeitet werden.

Die Trägermaterialien, die vorzugsweise aus einem elastischen Vliesstoff bestehen, können zumindest einen vermaschten elastischen Faden und/oder zumindest einen verwebten elastischen Faden aufweisen.

Derartige Trägermaterialien werden zur Herstellung von Strümpfen und Bandagen eingesetzt, die im allgemeinen aus einer räumlich begrenzten schlauchförmigen Maschenware bestehen. Vorteilhaft sind rundgestrickte beziehungsweise gewirkte Schlauchwaren aus elastischem Material, insbesondere Fasern oder Fäden. Als Grundlage dienen Ausgangsstoffe auf natürlicher und synthetischer Basis, so Polyester, Polyesteramide, - urethane, -olephine, -acrylate, -vinylverbindungen und Blockcopolymere, wie Styrolblockcopolymere, sowie native oder regenerierte Cellulose.

Auch starre Trägermaterialien, zum Beispiel Mulle, sind geeignet, um mit der erfindungsgemäßen Beschichtung ausgerüstet zu werden.

Aufgrund der Auswahl der Materialien, deren Aufbereitung und Weiterverarbeitung wird ein weites Feld an Produktmöglichkeiten abgedeckt. Eine anatomische Ausformung und Ausgestaltung entsprechend der gewünschten Einsatzgebiete und Indikationen ist möglich.

Die rutschhemmende Beschichtung kann vorteilhaft auf einer ein Körperteil vollständig umgebenden Bandage verwendet werden, wobei die Bandage insbesondere bei einer Dehnung von 10 % bis 350 % einen Kompressionsdruck von 3 bis 45 mm Hg, bevorzugt 8 bis 25 mm Hg, aufbauen sollte.

Die Bandage kann ein Strumpf sein, der einen von distal nach proximal abfallenden Druckverlauf aufweist, wobei in einer bevorzugten Ausführungsform im Bereich der Strumpföffnung die Haftung der Ausrüstung auf dem Strumpf mindestens 3 N/cm beträgt.
Der relative Abfall des Kompressionsdruckes sollte nicht mehr als 80 % betragen, bevorzugt 30 % bis 70 %. Der Strumpf hat dabei vorzugsweise eine Länge von weniger als 130 cm. Bevorzugt werden Strümpfe mit einer Länge von 30 bis 110 cm, besonders bevorzugt von 35 bis 95 cm. Der Umfang des Produktes im ausgedehnten Zustand beträgt bis zu 95 cm, bevorzugt weniger 90 cm, besonders bevorzugt von 16 bis 87 cm.

In einer ganz besonderen Ausführungsform erfolgt die Verwendung der Beschichtung auf einen im wesentlichen anatomisch geformten Kompressionsstrumpf, gebildet aus mindestens einem gemaschten elastischen Faden, wobei die rutschhemmende oder rutschverhindernde Beschichtung im oberen Öffnungsbereich des Strumpfes aufgetragen wird.
Der Kompressionsstrumpf kann eine Kompressionskraft von 15 bis 25 mm Hg im unteren Strumpfbereich und einer Kompressionskraft von 8 bis 15 mm Hg im oberen Strumpfbereich entwickeln und einen von proximal nach distal abfallenden Druckverlauf aufweisen.
Die rutschhemmende Ausrüstung besteht hier zu 95 bis 99,8 Gew.-% aus einem Polyaddukt auf Basis eines Polytetramethylenoxiddiol, 1,4 Butandiol und 4,'4 Diphenylmethandiisocyanat, wobei der Gewichtsanteil gebildet aus Polytetramethylenoxiddiol und 4,4'Diphenytmethadiisocyanat größer 78 Gew.-% ist und das Stoffmengenverhältnis von 4,'4 Diphenylmethandiisocyanat zu der Summe aus Polytetramethylenoxiddiol und 1,4 Butandiol zwischen und 0,96 und 1,01 beträgt.

Vorteilhaft ist, wenn mit der Beschichtung versehene Träger, wie Bandagen oder Strümpfe, steril sind.

Die Beschichtung der rutschhemmenden oder rutschverhindernden Ausrüstung kann im direkten Verfahren sowie im Transferverfahren aufgebracht werden. Vorteilhaft ist das Aufbringen aus der Schmelze, hierbei entfällt das Entfernen von Hilfsmitteln, wie es bei der Beschichtung aus Lösungen oder Dispersionen der Fall ist.

Die Herstellung solcher Polyaddukte ist bekannt, so erfolgt diese nach dem Batchverfahren oder in kontinuierlichen Herstellungsverfahren. Beispielhaft sind Extrusionsverfahren oder Gurtbandgießverfahren für Ansätze im Produktionsmaßstab zu nennen. Die Herstellung kann in einem oder in mehreren Schritten durchgeführt werden.

Im folgenden seien beispielhaft bevorzugte Verfahren zur Aufbringung der Beschichtung beschrieben, ohne damit die Erfindung unnötig einschränken zu wollen. Des weiteren wird ein erfindungsgemäß ausgerüsteter Strumpf gezeigt.

### Beispiele

Ohne hohen Verfahrensaufwand lassen sich die Polyaddukte im 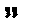 One-Shot-Verfahren" herstellen. Besonders vorteilhaft ist die Darstellung von Isocyanat-vernetzten oligomeren Diolen. Es lassen sich aliphatische und aromatische Isocyanate mit zwei oder drei endständigen funktionellen Gruppen verwenden.

Mit Blick auf Kosten und Herstellung wurden beispielhaft Versuche auf Basis von 4,4' Diphenylmethandiisocyanat beschrieben. Für den Fachmann bedarf es keiner erfinderischen Tätigkeit, bei der Verwendung von anderen Rohprodukten andere Versuchseinstellungen zu finden.

Es wurde das verwendete oligomere Polyol (E1) im Umluftofen geschmolzen und auf 84 °C Starttemperatur gebracht. Nach dem Erreichen dieser Temperatur wurde das Edukt (E2) und das ebenfalls geschmolzene und auf 50 °C temperierte Edukt (E3) zugegeben. Das Mischen wurde bis zum Erreichen der Gießtemperatur bei ca. 120 °C fortgesetzt. Anschließend wurde die Reaktionsmischung in eine teflonbeschichtete Wanne gegossen und auf einem Heiztisch bis zur 10. Minute nach dem Reaktionsbeginn bei 120 °C gehalten. Die Mischtemperatur hängt vom Hartsegmentanteil ab. Die Temperung erfolgte bei 85 °C über eine Dauer von 16 Stunden im Umluftofen. Nach dem Mahlen wurde das gewonnene Granulat nochmals 5 Stunden bei 110 °C getempert.
Zur Verkürzung des Tempervorganges kann das Polyaddukt auch in einem Extruder aufbereitet und granuliert werden.

Wichtig für die Waschbeständigkeit des Polyadduktes ist die Vermeidung von Nebenreaktionen an der Seitenkette, da diese hydrolyseempfindlich sind. Für die Reaktionen mit Isocyanaten sollte daher ein geringer Anteil an Allophanatgruppen im sonst weitestgehend linearen Polymer erreicht werden. Der Anteil der Allophanatgruppen sollte maximal 5 Gew.-% sein. Bevorzugt sind Allophanatgruppenanteile von weniger als 2 Gew.-%, besonders bevorzugt sind Anteile von weniger als 0,5 Gew.-%. Der Anteil der reaktiven Gruppen des Eduktes E3 sollte nach dem Abschluß der Vernetzung insgesamt kleiner 0,5 %, bevorzugt kleiner 0,3 % sein.

### Rezepturen der Versuchsreihe I

| | Einwaage | | | | |
|---|---|---|---|---|---|
| Rohstoffe | A | B | C | D | E |
| Polyether 1 | 129,32 | 128,57 | | | |
| Polyether 2 | | | | 134,61 | |
| Polyether 3 | | | 162,72 | | 144,47 |
| MDI | 60,09 | 60,84 | 31,63 | 53,85 | 45,03 |
| 1,4 Butandiol | 10,59 | 10,59 | 5,64 | 11,54 | 10,50 |
| Bezugsmasse | 200 | 200 | 200 | 200 | 200 |

### Rezepturen der Versuchsreihe II

| | Einwaage | | |
|---|---|---|---|
| Rohstoffe | F | G | H |
| Polyether 4 | 135,9 | | |
| Polyester 1 | | 129 | 143,61 |
| MDI | 54,24 | 60,5 | 51,06 |
| 1,4 Butandiol | | 10,5 | 5,32 |
| 1,6 Hexandiol | 9,86 | | |
| Bezugsmasse | 200 | 200 | 200 |

Der Wassergehalt im oligomeren Diol (E1) und im Edukt (E2) betrug weniger als 0,3 %. Hydroxylzahl der oligomeren Polyole ist in der nachfolgenden Tabelle beschrieben.

| | Diol | | | | |
|---|---|---|---|---|---|
| | Polyether 1 | Polyether 2 | Polyether 3 | Polyether 4 | Polyester 1 |
| Hydroxylzahl | 112,9 | 73 | 46,3 | 112 | 115 |

Für die erfindungsgemäße Ausrüstung ist es notwendig, die rutschhemmende Substanz auf das Trägermaterial zu beschichten. Dieses kann direkt oder durch Transferierung erfolgen. Die Beschichtung kann mit oder ohne Unterbrechung durchgeführt werden.
Prinzipiell können beschichtungsfreie Stellen oder aber auch nur partielle Beschichtungsstellen hergestellt werden. Dieses ist über die üblichen Beschichtungsverfahren wie Sprühdruck, insbesondere Raster-, Gravur- und Siebdruck möglich. Eine weitere Möglichkeit ist das Ausstanzen, Fräsen oder Schneiden einer vollflächigen Beschichtung mit dem anschließenden Transferieren der rutschhemmenden oder -hindernden Substanz.

### Beispiel 1

Ein anatomisch geformter Strumpf mit einem Kompressionsdruck von 12 mm Hg an der oberen Öffnung wurde durch Vermaschen von Nylonfäden hergestellt. Die Beschichtung im Öffnungsbereich erfolgte bei einer Temperatur von 165 °C. Es wurden acht Streifen von 6 mm x 30 mm V-förmig beschichtet. Der Masseauftrag wurde auf ein Quadratmeter umgerechnet und betrug 500 g/m².

Als rutschhemmende Substanz wurde ein vernetztes oligomeres Diol auf Polyetherbasis verwendet. Als weitere Edukte wurden 1,4 Butandiol und 4',4-Diphenylmethandiisocyanat verwendet. Die Hydroxylzahl des oligomeren Diols betrug 73 mg KOH/g. Der Wassergehalt des 1,4 Butandiols und des oligomeren Diols wurde nach Karl-Fischer bestimmt. Der Wassergehalt betrug weniger als 0,2 %.

Es wurde das verwendete oligomere Polyol (E1) im Umluftofen geschmolzen und auf 84 °C Starttemperatur gebracht. Nach dem Erreichen dieser Temperatur wurde das Edukt (E2) und das ebenfalls geschmolzene und auf 50 °C temperierte Edukt (E3) zugegeben. Das Mischen wurde bis zum Erreichen der Gießtemperatur bei ca. 120 °C fortgesetzt. Anschließend wurde die Reaktionsmischung in eine teflonbeschichtete Wanne gegossen und auf einem Heiztisch bis zur 10. Minute nach dem Reaktionsbeginn bei 120 °C gehalten. Die Mischtemperatur hängt vom Anteil der Phase 1 ab. Die Temperung erfolgte bei 85 °C über eine Dauer von 16 Stunden im Umluftofen. Nach dem Mahlen wurde das gewonnene Granulat nochmals 5 Stunden bei 110 °C getempert.

| | Einwaage |
|---|---|
| Rohstoffe | D |
| Polyether 1 | |
| Polyether 2 | 134,61 |
| Polyether 3 | |
| MDI | 53,85 |
| 1,4 Butandiol | 11,54 |
| Bezugsmasse | 200 |

Der Rest Isocyanatgehalt betrug weniger als 0,1 %. Der Anteil der ersten Phase hatte einen Gewichtsanteil von 78 Gew.-% und das Stoffmengenverhältnis von E 3 zu E1+E2 betrug 0,998.

Das aufgetragene rutschhemmende Polyaddukt ist sehr anschmiegsam. Nach zehn Waschgängen bei 95 °C zeigte es eine nahezu unveränderte rutschhemmende Wirkung. Ein Ablösen oder Auflösen der Beschichtung wurde nach den Waschgängen nicht festgestellt. Die rutschhemmende Ausrüstung ist silikonfrei und nicht klebrig.

## Patentansprüche

1. Rutschhemmende Beschichtung zur zumindest partiellen Aufbringung auf den Körper bedeckende Trägermaterialien, beinhaltend ein Polyaddukt mit einer Phase 1 und einer Phase 2, wobei der Masseanteil von Phase 1 größer 60 Gew.-% ist,
Phase 1 ein Polyadditionsprodukt aus mindestens einem Edukt E1 und mindestens einem Edukt E3 ist, wobei Edukt E1 ein oligomeres Diol darstellt, welches eine Hydroxylzahl von mehr als 20 mg KOH/g (Diol) aufweist, und wobei Edukt E3 ein silikonfreies, zumindest bifunktionelles Reaktionsedukt mit einem Molekulargewicht von weniger als 300 g/mol ist,
Phase 2 ein Polyadditionsprodukt aus mindestens einem Edukt E2 und mindestens einem Edukt E3 ist, wobei das Edukt E2 wenigstens teilweise endständige funktionelle Gruppen aufweist und
die Beschichtung ein Stoffmengenverhältnis der zu vernetzenden Edukte E3 zu der Summe aus einem oder mehreren oligomeren Diolen (Edukt E1) und dem Anteil weiterer Edukte E2 einen Quotienten von 0,9 bis 1,1 aufweist.

2. Rutschhemmende Beschichtung nach Anspruch 1, dadurch gekennzeichnet, daß die Trägermaterialien zumindest einen vermaschten elastischen Faden und/oder zumindest einen verwebten elastischen Faden beinhalten.

3. Rutschhemmende Beschichtung nach den Ansprüchen 1 und 2, dadurch gekennzeichnet, daß die Trägermaterialien aus einem elastischen Vliesstoff bestehen.

4. Rutschhemmende Beschichtung nach den Ansprüchen 1 bis 3, dadurch gekennzeichnet, daß die Beschichtung im Temperaturbereich von 5 °C bis 100 °C hydrolysebeständig ist.

5. Rutschhemmende Beschichtung nach mindestens einem vorangegangenen Anspruch, dadurch gekennzeichnet, daß die Beschichtung einen Schäumungsgrad von zumindest 5 Vol.-%, insbesondere 5 bis 85 Vol.-%, bevorzugt 10 bis 75 Vol.-%, besonders bevorzugt 20 bis 60 Vol.-% beträgt.

6. Rutschhemmende Beschichtung nach mindestens einem vorangegangenen Anspruch, dadurch gekennzeichnet, daß die Beschichtung weniger als 5 Gew.-%, bevorzugt 0,01 bis 2 Gew.-% Allophanatgruppen beinhaltet.

7. Rutschhemmende Beschichtung nach mindestens einem vorangegangenen Anspruch, dadurch gekennzeichnet, daß das Verhältnis vom Edukt 3 zu der Summe der Edukte 1 und Edukte 2 zwischen 0,91 und 1,05, besonders bevorzugt 0,92 bis 1,02 liegt.

8. Rutschhemmende Beschichtung nach mindestens einem vorangegangenen Anspruch, dadurch gekennzeichnet, daß der Gewichtsanteil der Phase 1 wenigstens 72 %, bevorzugt 75 bis 98 %, besonders bevorzugt 76 bis 95 %, beträgt.

9. Rutschhemmende Beschichtung nach mindestens einem vorangegangenen Anspruch, dadurch gekennzeichnet, daß das oligomere Diol ein Polyetherdiol, insbesondere ein Polytetramethylenoxiddiol oder Polyethylenoxiddiol oder deren Mischungen ist, eine Hydroxylzahl von 40 bis 230 mg KOH/g, bevorzugt 65 bis 224 mg KOH/g, besonders bevorzugt 70 bis 150 mg KOH/g, aufweist.

10. Rutschhemmende Beschichtung nach mindestens einem vorangegangenen Anspruch, dadurch gekennzeichnet, daß das Edukt E2 ein 1,2-Ethandiol, 1,4 Butandiol, 1,6 Hexandiol und/oder eine Mischung aus den vorgenannten ist.

11. Rutschhemmende Beschichtung nach mindestens einem vorangegangenen Anspruch, dadurch gekennzeichnet, daß das Edukt E3 ein aliphatisches oder aromatisches Diisocyanat ist.

12. Verwendung der rutschhemmenden Beschichtung auf einer ein Körperteil vollständig umgebenden Bandage, wobei die Bandage insbesondere bei einer Dehnung von 10% bis 350% einen Kompressionsdruck von 3 bis 45 mm Hg, bevorzugt 8 bis 25 mm Hg, aufbaut.

13. Verwendung nach vorangegangenem Anspruch 12, dadurch gekennzeichnet, daß die Bandage ein Strumpf ist, der einen von distal nach proximal abfallenden Druckverlauf aufweist.

14. Verwendung der rutschhemmenden Beschichtung nach mindestens einem vorangegangenen Anspruch, dadurch gekennzeichnet, daß im Bereich der Strumpföffnung die Haftung der Ausrüstung auf dem Strumpf mindestens 3 N/cm beträgt.

15. Verwendung der rutschhemmenden Beschichtung auf einem im wesentlichen anatomisch geformten Kompressionsstrumpf, gebildet aus mindestens einem gemaschten elastischen Faden, mit einer rutschhemmenden oder rutschverhindernden Beschichtung im oberen Öffnungsbereich des Strumpfes, mit einer Kompressionskraft von 15 bis 25 mm Hg im unteren Strumpfbereich und einer Kompressionskraft von 8 bis 15 mm Hg im oberen Strumpfbereich, einen von proximal nach distal abfallenden Druckverlauf aufweist, dadurch gekennzeichnet, daß die rutschhemmende Ausrüstung zu 95 bis 99,8 Gew.-% ein Polyaddukt auf Basis eines Polytetramethylenoxiddiol, 1,4 Butandiol und 4,'4 Diphenylmethandiisocyanat besteht, wobei der Gewichtsanteil gebildet aus Polytetramethylenoxiddiol und 4,4' Diphenylmethandiisocyanat größer 78 Gew.-% ist und das Stoffmengenverhältnis von 4,4' Diphenylmethandiisocyanat zu der Summe aus Polytetramethylenoxiddiol und 1,4 Butandiol zwischen und 0,96 und 1,01 beträgt.
